(19) Europäisches Patentamt
European Patent Office
Office européen des brevets

(11) EP 3 998 611 A1

(12) EUROPEAN PATENT APPLICATION
published in accordance with Art. 153(4) EPC

(43) Date of publication:
18.05.2022  Bulletin 2022/20

(51) International Patent Classification (IPC):
G16B 40/00 (2019.01)        G16B 25/10 (2019.01)
G16B 50/00 (2019.01)        G16H 20/00 (2018.01)

(21) Application number: 20837519.6

(52) Cooperative Patent Classification (CPC):
G16B 25/10; G16B 40/00; G16B 50/00;
G16H 20/00

(22) Date of filing: 07.07.2020

(86) International application number:
PCT/KR2020/008843

(87) International publication number:
WO 2021/006596 (14.01.2021 Gazette 2021/02)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority:  10.07.2019  KR 20190083016

(71) Applicants:
• Korea Advanced Institute of Science and
Technology
Daejeon 34141 (KR)
• Pentamedix Co., Ltd.
Sujeong-gu, Seongnam-si, Gyeonggi-do 13449
(KR)

(72) Inventors:
• CHOI, Jung Kyoon
Daejeon 34141 (KR)
• JANG, Kiwon
Daejeon 34141 (KR)
• CHO, Dae Yeon
Seongnam-si Gyeonggi-do 13540 (KR)

(74) Representative: dompatent von Kreisler Selting
Werner -
Partnerschaft von Patent- und Rechtsanwälten
mbB
Deichmannhaus am Dom
Bahnhofsvorplatz 1
50667 Köln (DE)

(54) MACHINE LEARNING MODEL-BASED ESSENTIAL GENE IDENTIFICATION METHOD AND ANALYSIS APPARATUS

(57) A machine learning model-based essential gene identification method comprises the steps of: receiving, by an analysis apparatus, inputs of expression pattern information on genes of a specific cell; inputting, by the analysis apparatus, the expression pattern information to a machine learning model; and determining, by the analysis apparatus, whether a target gene from among the genes is essential in the survival of the cell on the basis of information output by the machine learning model. The machine learning model comprises parameters which are trained based on a training data set, and the training data set comprises data for the gene expression of the specific cell and label values for whether the specific cell dies.

FIG. 1

**Description**

[Technical Field]

Following description relate to a technique for identifying genes essential for survival of a specific cell based on a transcriptome pattern of the specific cell.

[Background Art]

Ribonucleic acid interference (RNAi) and clustered regularly interspaced short palindromic repeats (CRISPR) techniques may knockdown or knockout an expression of a specific gene to determine whether the specific gene is essential for cell survival. The techniques are described as RNAi/CRISPR screens. For example, the RNAi/CRISPR screens may identify genes essential for tumor cells.

[Disclosure]

[Technical Problem]

However, ribonucleic acid interference (RNAi)/clustered regularly interspaced short palindromic repeats (CRISPR) screens can only be analyzed in an in vitro cellular environment. Therefore, there are limitations in that the RNAi/CRISPR screens consume a great deal of time and a high cost.
Technologies be described below are to provide a method of identifying essential genes of a cell in-silico based on data for a gene expression of cells.

[Technical Solution]

A machine learning model-based essential gene identification method includes receiving, by an analysis apparatus, expression pattern information on a gene of a specific cell, inputting, by the analysis apparatus, the expression pattern information to a machine learning model, and determining, by the analysis apparatus, whether a target gene among the genes is essential in survival of the cell on the basis of information output by the machine learning model.
A machine learning model-based tumor cell-specific essential gene identification method includes receiving, by the analysis apparatus, data for a gene expression of each of a normal cell and a tumor cell of the same target, inputting, by the analysis apparatus, first gene expression pattern information, in which an expression of a target gene to be analyzed is regulated for the tumor cell, to a machine learning model to generate a first value, inputting, by the analysis apparatus, second gene expression pattern information, in which an expression of the same gene as the target gene is regulated for the normal cell, to the machine learning model to generate a second value, and comparing, by the analysis apparatus, the first value with the second value to determine whether the target gene is an essential gene specific to the tumor cell.
An analysis apparatus for selecting a machine learning model-based essential gene includes an input device configured to receive expression data for cellular genes, a storage device configured to store a machine learning model that receives a gene expression pattern in which an expression of a specific gene is regulated and outputs essentiality information on the specific gene, and a processor configured to input a gene expression pattern for the cell, in which an expression of a target gene is regulated in the expression data input from the input device, to the machine learning model, and determine essentiality of the target gene based on a value output by the machine learning model.
The machine learning model includes a parameter trained based on a training data set, and the training data set includes data for the gene expression of the specific cell and a label value for whether the specific cell dies.

[Advantageous Effects]

Technologies to be described below can identify essential genes of cells in a short time and at low cost using a machine learning model. Technologies to be described below can be utilized for neoantigen screening by selecting essential genes of tumor cells.

[BRIEF DESCRIPTION OF THE DRAWINGS]

FIG. 1 illustrates an example of a system for identifying essential genes of a specific cell.

FIG. 2 illustrates an example of a schematic process of identifying an essential gene in an analysis apparatus.

FIG. 3 illustrates an example illustrating a process of identifying an essential gene based on a perturbed gene expression.

FIG. 4 illustrates another example illustrating a process of identifying an essential gene based on the perturbed gene expression.

FIG. 5 illustrates an example of a process of training a deep learning model.

FIG. 6 illustrates an example of a process of predicting an essential gene using the deep learning model.

FIG. 7 illustrates an example of a computing device for predicting essential genes of a cell using a deep learning model.

FIG. 8 illustrates an example of an analysis apparatus for identifying an essential gene.

FIG. 9 illustrates an experimental result verifying an effect of the deep learning model.

[Best Mode]

The present disclosure may be variously modified and have several exemplary embodiments. Therefore, specific exemplary embodiments of the present disclosure will be illustrated in the accompanying drawings and be described in detail. However, it is to be understood that the present invention is not limited to a specific exemplary embodiment but includes all modifications, equivalents, and substitutions without departing from the scope and spirit of the present invention.

Terms such as "first,", "second,", "A," "B," and the like may be used to describe various components, but the components are not to be interpreted to be limited to the terms and are used only for distinguishing one component from other components. For example, a "first" component may be named a "second" component and the "second" component may also be similarly named the "first" component, without departing from the scope of the present disclosure. A term "and/or" includes a combination of a plurality of related described items or any one of the plurality of related described items.

It should be understood that the singular expression includes the plural expression unless the context clearly indicates otherwise, and it will be further understood that the terms "comprises" or "have" used in this specification specify the presence of stated features, steps, operations, components, parts, or a combination thereof but do not preclude the presence or addition of one or more other features, numerals, steps, operations, components, parts, or a combination thereof.

Prior to the detailed description of the drawings, it is to be clarified that the components in this specification are only distinguished by the main functions of each component. That is, two or more components to be described below may be combined into one component, or one component may be divided into two or more components for each subdivided function. In addition, each of the constituent parts to be described below may additionally perform some or all of the functions of other constituent parts in addition to the main functions of the constituent parts, and some of the main functions of the constituent parts may be performed exclusively by other components.

In addition, in performing the method or the operation method, each of the processes constituting the method may occur differently from the specified order unless a specific order is explicitly described in context. That is, the respective steps may be performed in the same sequence as the described sequence, performed at substantially the same time, or performed in an opposite sequence to the described sequence.

Hereinafter, key terms used in the description will be described.

A cell is a sample acquired from an individual to be analyzed or a specific tissue of the individual and may refer to a cell line, a group of cells, or a single cell. The object is basically acquired from a human being. However, the individual is not necessarily limited to a human being.

A transcriptome refers to a set of expressed ribonucleic acids (RNAs) present in a cell, a group of cells, or an individual.

Essential genes or dependent genes refer to a gene essential for proliferation or survival of cells. The essential genes are genes which result in cell death when expressions of the essential genes are knocked-down or knocked-out. Universally essential genes refer to genes that are universally essential for the survival of various types of tumors or tumor cells. Cancer patient-specific essential genes are genes that are specifically essential for the survival of tumor cells derived from individual cancer patients. Hereinafter, the essential genes refer to universally essential genes and/or cancer patient-specific essential genes. Hereinafter, for convenience of description, a tumor will be mainly described.

Machine learning or learning is a field of artificial intelligence and refers to a field of algorithms developed so that a computer may be trained. A machine learning model or a learning model refers to a model developed so that a computer may be trained. There are various models such as an artificial neural network and a decision tree depending on the approach to the learning model. Hereinafter, for convenience of description, a deep learning model will be mainly described.

The analysis apparatus is an apparatus that identifies essential genes of cells using the learning model. The analysis apparatus processes and analyzes genome data using the installed program. The analysis apparatus is an apparatus such as a smart device (smartphone and tablet), a computer device (personal computer (PC) and laptop), a server, or an analysis-only chipset.

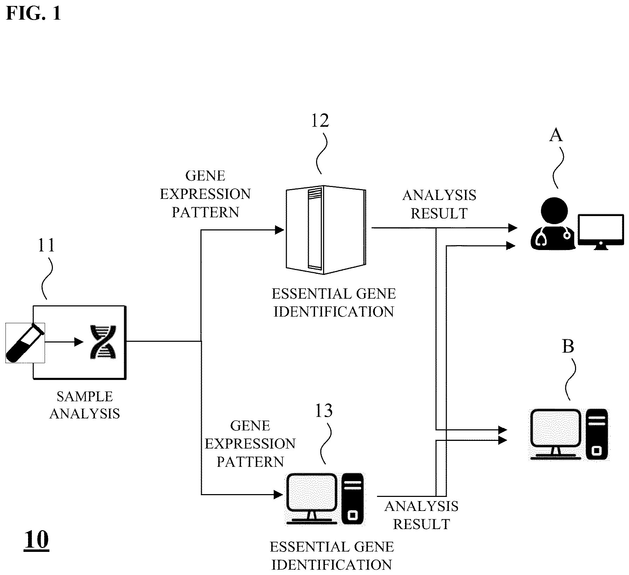

FIG. 1 illustrates an example of a system 10 for identifying essential genes of a specific cell.

A transcriptome processing device 11 generates gene expression information by analyzing cells. The transcriptome processing device 11 may acquire cellular gene expression information using techniques such as RNA sequencing (RNA-Seq) and DNA microarray.

In FIG. 1, the analysis apparatus shows two types. The analysis apparatus 12 is a server connected through a network. The analysis apparatus 13 is a computer device such as a PC. The analysis apparatus 12 or 13 receives a cellular gene expression pattern. The gene expression pattern includes information on an expression of each gene. The analysis apparatus 12 or 13 identifies essential genes in the cell by inputting the gene expression pattern to a learning model.

The analysis apparatus 12 or 13 may provide an analysis result to researcher A. Alternatively, the analysis apparatus 12 or 13 may provide an analysis result to another analysis apparatus B that performs additional analysis using information on essential genes. For example, another analysis apparatus B may identify neoantigens using essential genetic information along with tumor cell-specific mutation information.

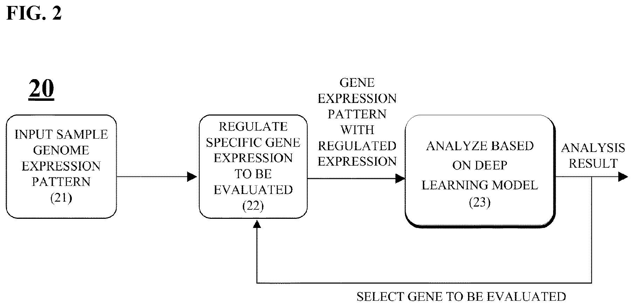

FIG. 2 illustrates an example of a schematic process of identifying an essential gene in an analysis apparatus (20). The analysis apparatus receives a genome expression pattern of a cell (21). The analysis apparatus selects a specific gene to be evaluated. For example, the analysis apparatus may select a $k^{th}$ gene from among the gene set. The $k^{th}$ gene to be evaluated is referred to as a target gene. The analysis apparatus regulates an expression of the $k^{th}$ gene (22). For example, the analysis apparatus may knockdown the expression of the $k^{th}$ gene.

The analysis apparatus may convert the regulated genome expression pattern into an input value of a deep learning model. The analysis apparatus may convert the genome expression pattern into a vector value. The genome expression pattern is information on an expression of consecutive genes. Therefore, the genome expression pattern may be expressed as a one-dimensional vector sequence. The vector sequence includes an order of a gene sequence and information on the expression of the corresponding gene.

The analysis apparatus may input the vector sequence of the gene expression pattern to the deep learning model. The analysis apparatus inputs the cellular gene expression pattern, in which the expression of the $k^{th}$ gene is regulated, to the deep learning model and analyzes the cellular gene expression pattern (23). The deep learning model outputs the analysis result indicating whether the $k^{th}$ gene is an essential gene in the cell.

The analysis apparatus may select other genes to be evaluated and analyze whether the genes are essential genes by repeating the same process. For example, the analysis apparatus selects a $l(k \neq l)^{th}$ gene and knocks-down an expression of a $l^{th}$ gene in an original gene expression pattern input in operation 21. The analysis apparatus inputs and analyzes the gene expression pattern, in which the expression of the $l^{th}$ gene is regulated, to the deep learning model and analyzes the gene expression pattern.

The deep learning model used to classify essential genes will be described. The deep learning model receives the cellular gene expression information and outputs information on whether the cells die. The process of training the deep learning model will be described. The training data set includes gene expression information (input value) of a specific reference and information (label value) on whether a reference cell having the corresponding expression dies. As the training data, experimentally confirmed data may be used.

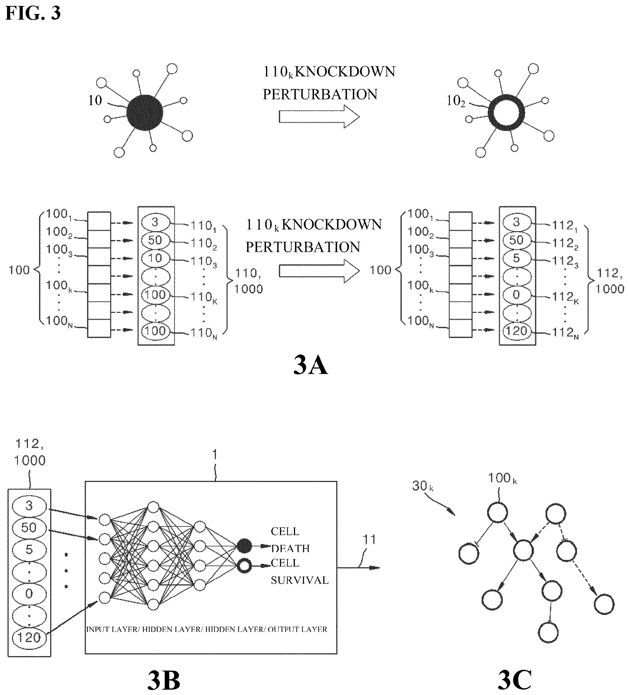

FIG. 3 illustrates an example illustrating a process of identifying an essential gene based on a perturbed gene expression. FIG. 3 illustrates an example of a process for identifying essential genes of a tumor cell.

FIG. 3A is a diagram illustrating an expression of tumor cellular genes and a perturbed expression of tumor cellular genes. FIG. 3B is a diagram for describing a structure according to an embodiment of a prediction model that receives expressions of cellular genes and outputs a probability of cell death. FIG. 3C conceptually illustrates a $k^{th}$-gene regulation network $30_k$ including a $k^{th}$-gene 100k of a tumor cell 10. The gene regulation network will be described below.

Referring to FIG. 3A, the tumor cell 10 of a cancer patient may include N genes 100.

Perturbation that knocks-down the expression $110_k$ of the $k^{th}$-gene in a $k^{th}$-gene regulation network 30k including the $k^{th}$-gene 100k of the tumor cell 10 can be simulated. Simulation of such perturbation is possible in various ways using the related art, and a specific method for simulation of such perturbation does not limit the scope of the present invention.

A perturbed-tumor cell 102 refers to a tumor cell in a state in which a perturbation has occurred in the tumor cell 10. In FIG. 3A, squares arranged consecutively in a vertical direction represent genes of each of the tumor cell 10 or the perturbed-tumor cell 102. The $k^{th}$ gene is denoted by reference number 100k using the subscript k. Here, k may be a natural number of one or more, i.e., k=1, 2, 3,..., or N.

In FIG. 3A, expressions of the genes of the tumor cell 10 are denoted by reference number 110. Expressions of genes of the perturbed-tumor cells 102 are denoted by reference number 112. In FIG. 3A and other drawings presented below, expressions of genes of any cell or a cell line are collectively denoted by reference number 1000.

The expressions 112 of a set of genes 100 of the perturbed-tumor cell 102 may be regarded as a $k^{th}$-set input value input to a deep learning model 1 to be described below.

In FIG. 3A, numbers presented inside circles consecutively arranged in the vertical direction indicate the expression of the corresponding gene as a number.

As illustrated in FIG. 3A, it may be confirmed that the expressions of the genes are changed when the perturbation that knocks-down the expression $110_k$ of the $k^{th}$-gene occurs.

FIG. 3B illustrates an example of a deep learning model 1. The deep learning model 1 may be a neural network including an input layer, hidden layers, and an output layer. When the $k^{th}$-set input value is input to the input layer of the deep learning model 1, two probability values may be output to the output layer. The sum of the two output values may be one or less. One of the two probability values indicates the probability that the cell will reach death, and the other indicates the probability that the cell will grow. Alternatively, the deep learning model 1 may output a single piece of information on cell survival or cell death.

An output value output by the deep learning model 1 may be indicated by reference number 11. The output value 11 may include one or more of the probability that the tumor cell will die and the probability that the tumor cell will grow.

The analysis apparatus may include determining whether the $k^{th}$-gene is an essential gene of the tumor cell based on the probability of the death of the tumor cell. For example, when the probability of the death of the tumor cell is greater than or equal to a predetermined threshold (for example, 0.8), the analysis apparatus may determine that the $k^{th}$-gene is the essential gene of the tumor cell, and when the probability of the death of the tumor cell is less than the predetermined threshold value, the analysis apparatus may determine that the $k^{th}$-gene is not the essential gene.

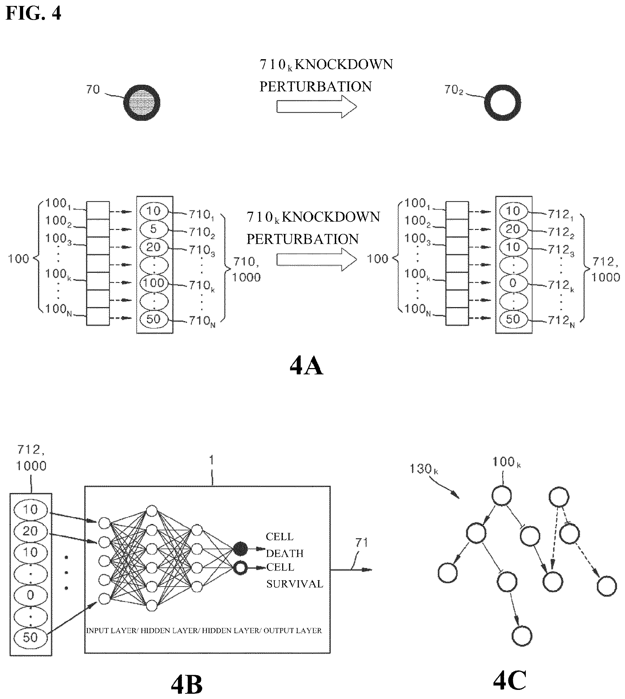

FIG. 4 illustrates an example illustrating a process of identifying essential genes based on a perturbed gene expression. FIG. 4 illustrates an example of a process for identifying essential genes in a normal cell.

FIG. 4A is a diagram illustrating expressions of normal cellular genes and expressions of perturbed normal cellular genes.

FIG. 4B is a diagram for describing a structure according to an embodiment of a prediction model that receives expressions of cellular genes and outputs a probability of cell death.

FIG. 4C conceptually illustrates a $k^{th}$-gene regulation network $130_k$ including a $k^{th}$-gene 100k of a normal cell 70.

The $k^{th}$-gene regulation network $130_k$ illustrated in FIG. 4C conceptually indicates the gene regulation network $130_k$ in the normal cell 70 and may be different from the $k^{th}$-gene regulation network $30_k$ of the tumor cell 10 illustrated in FIG. 3.

When described with reference to FIG. 4A, the normal cell 70 of a cancer patient may include N genes 100.

Perturbation that knocks-down an expression $710_k$ of the $k^{th}$-gene in the $k^{th}$ gene regulation network $130_k$ including the $k^{th}$-gene 100k of the normal cell 70 may be simulated.

A perturbed-normal cell 702 refers to a normal cell in a state in which the perturbation has occurred in the normal cell 70.

In FIG. 4A, squares arranged consecutively in a vertical direction indicate the genes of each of the normal cell 70 or the perturbed-normal cell 702. The $k^{th}$ gene is denoted by reference number 100k using the subscript k. Here, k may be a natural number of one or more, i.e., k=1, 2, 3,..., or N.

In FIG. 4A, expressions of the genes in the normal cell 70 are indicated by reference number 710, and expressions of the genes of the perturbed-normal cell 702 are indicated by reference number 712. In FIG. 4A and other diagrams including the same, expressions of genes in any cell or a cell line are collectively indicated by reference number 1000.

The expressions 712 of a set of genes 100 of the perturbed-normal cell 702 may be regarded as a $k^{th}$-set input value input to the deep learning model 1 to be described below.

In FIG. 4A, numbers presented inside circles consecutively arranged in the vertical direction indicate the expression of the corresponding gene as a number.

As illustrated in FIG. 4A, it may be confirmed that the expressions of the genes are changed when the perturbation that knocks-down the expression $710_k$ of the $k^{th}$-gene occurs.

The deep learning model 1 illustrated in FIG. 4B may be the same neural network as illustrated in FIG. 3B.

The output value output by the deep learning model 1 may be indicated by reference number 71. The output value 71 may include one or more of the probability that the normal cell will die and the probability that the normal cell will grow.

The analysis apparatus may determine whether the $k^{th}$-gene is an essential gene of the normal cell based on the output value 71, that is, the probability of the death of the normal cell. For example, when the probability of the death of the normal cell is greater than or equal to a predetermined threshold (for example, 0.8), the analysis apparatus may determine that the $k^{th}$-gene is the essential gene of the normal cell, and when the probability of the death of the normal cell is less than the predetermined threshold value, the analysis apparatus may determine that the $k^{th}$-gene is not the essential gene.

The analysis apparatus may also determine an essential gene specific to the tumor cell by using both the information on the gene determined to be the essential gene of the tumor cell and the information on the gene determined to be the essential gene of the normal cell.

For example, the analysis apparatus may determine whether the $k^{th}$-gene 100k is an essential gene specific to the tumor cell 10 based on the probability 11 of the death of the tumor cell 10 and the probability 71 of the death of the normal cell 70 with respect to the $k^{th}$-gene 100k.

When the expression of the $k^{th}$-gene 100k is suppressed and when it is determined that both the probability 11 of the death of the tumor cell 10 and the probability 71 of the death of the normal cell 70 are greater than or equal to the threshold value, the analysis apparatus may determine that the $k^{th}$-gene 100k is not an essential gene specific to the tumor cell 10. That is, when the $k^{th}$-gene 100k is determined to be an essential gene of both the tumor cell 10 and the normal cell 70, the analysis apparatus may determine that the $k^{th}$-gene 100k is not an essential gene specific to the tumor cell 10.

On the other hand, when the expression of the $k^{th}$-gene 100k is suppressed and when it is determined that the probability 11 of the death of the tumor cell 10 is greater than or equal to the threshold value but the probability 71 of the death of the normal cell 70 is less than or equal to the threshold value, the analysis apparatus may determine that the $k^{th}$-gene 100k is an essential gene specific to the tumor cell 10. That is, when it is determined that the $k^{th}$-gene 100k is an essential gene of the tumor cell 10 but is not an essential gene of the normal cell 70, the analysis apparatus may determine that the $k^{th}$gene 100k is an essential gene specific to the tumor cell 10.

When it is determined that the $k^{th}$-gene 100k is an essential gene specific to the tumor cell 10, by knocking-down the expression of the $k^{th}$-gene 100k, it is highly likely that the tumor cell 10 is led to die, and the normal cell 70 continues to survive.

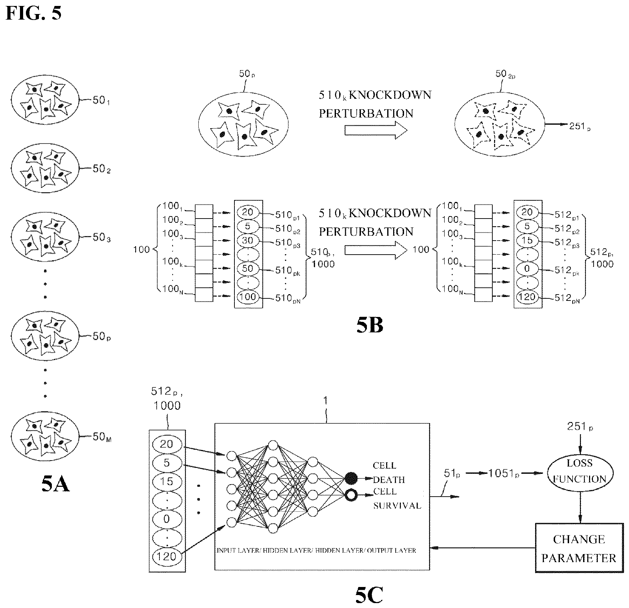

FIG. 5 illustrates an example of a process of training a deep learning model. The deep learning model may have a structure different from that illustrated in FIG. 5.

FIG. 5A illustrates a representation of M cell lines. A $p^{th}$ cell line is denoted by reference number 50p using the subscript p. In this case, p may be a natural number having a value of 1, 2, 3,..., or M.

FIG. 5B illustrates an example of perturbing a gene expression for the $p^{th}$ cell line. The gene expression may be controlled experimentally using techniques such as ribonucleic acid interference (RNAi) and clustered regularly interspaced short palindromic repeats (CRISPR). Therefore, the input value may use actually experimentally measured data. Furthermore, the gene expression may be constantly perturbed in-silico. A model of changing a gene expression in-silico is referred to as a gene regulation network. The gene regulation network will be described below.

The gene regulation network may perform perturbation that knocks-down an expression $510_k$ of the $k^{th}$-gene 100k of a $p^{th}$-cell line 50p. The input value becomes an expression $512_p$ of a set of genes 100 of a perturbed cell line $50_{2p}$. In FIG. 5, a gene set is represented by a square box, and the gene expression in the gene set is represented by a circle. The expression of the entire gene set was denoted by 1000.

FIG. 5C illustrates an example of a process of training the deep learning model 1.

The deep learning model 1 may include the above-described layers therein and nodes included in the layers, and links representing a signal flow between the nodes. Weights of the links may be regarded as parameters included in the deep learning model 1.

The deep learning model 1 may include a process of repeatedly executing a process of updating values of the parameters. The process of updating parameters may be performed on a specific gene of a specific cell line. That is, the deep learning model 1 may be trained once using the expressions of each gene obtained by applying a perturbation that suppresses the expression of the specific gene of the specific cell line. When the above-described M cell lines each include N genes, the parameters of the deep learning model 1 may be updated and trained at least M*N times.

The expression values of the genes 100 of the $p^{th}$-cell line 50p and a $p^{th}$reference value 251p indicating whether the gene is an essential gene may be prepared. In this case, the $p^{th}$-reference value 251p may be obtained from essential gene results experimentally observed by suppressing the genes 100 of the $p^{th}$-cell line 50p through the RNAi and CRISPR techniques.

The deep learning model 1 may receive $p^{th}.k^{th}$-set input values 512p and output a probability 51p for death of the $p^{th}$-cell line 50p.

A computer device for constructing a deep learning model may calculate a $p^{th}$determination value 105lp indicating whether the $k^{th}$-gene 100k is an essential gene of the $p^{th}$-cell line 50p based on the probability 51p for the death of the $p^{th}$-cell line 50p. The computer device may update the parameters of the deep learning model 1 to reduce a difference value between the $p^{th}$-determination value 1051p and the $p^{th}$-reference value 251p. The deep learning model 1 is trained by repeating the process of updating parameters in this way.

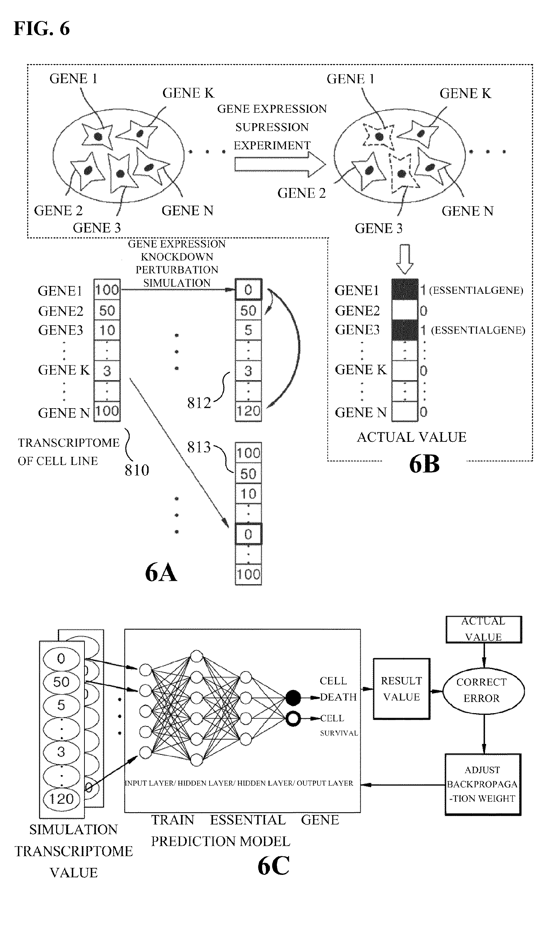

FIG. 6 illustrates another example of a process of training a deep learning model.

FIG. 6A illustrates a transcriptome of a cell line. The cell line may include N genes, and regions divided by squares in FIG. 6A represent different genes. Numbers given for each gene indicate expressions of each gene.

Transcriptome expressions 810 of genes 1 to N of the corresponding cell line are as illustrated in FIG. 6A. The analysis apparatus may regulate a gene expression of a gene to be analyzed by using a gene regulation network. FIG. 6A illustrates an example in which gene expressions of gene 1 and gene k are each knocked-down.

FIG. 6A illustrates expressions 812 of genes of a cell line that may be obtained when the analysis apparatus simulates a perturbation that knocks-down the expression of the gene 1. In this case, it may be confirmed that the expression of the gene 1 was naturally knocked-down, and the expressions of other genes were also changed. When the expression of the gene 1 is knocked-down, an expression of gene 3 is knocked-down and an expression of gene N is knocked-up.

FIG. 6A illustrates expressions 813 of genes of a cell line that may be obtained when the analysis apparatus simulates a perturbation that knocks-down the expression of the gene k. In this case, the expression of the gene k is knocked-down, but expressions of other genes are not knocked-down.

FIG. 6A illustrates the results of reducing the expressions of the gene 1 and gene k, but the analysis apparatus may also regulate the expressions of other genes for which essentiality is to be evaluated and input the regulated expressions to the deep learning model.

FIG. 6B illustrates information indicating whether each gene of a cell line is an essential gene leading to the cell line death. The information may be acquired from results of experiments on a relationship between gene expression knockdown and cell line death for a specific gene. Regions divided by squares in FIG. 6B represent different genes. In FIG. 6B, a black rectangle represents an essential gene, and a white rectangle represents a non-essential gene. Numbers shown on the right side of each square in FIG. 6B have a value of 1 (black) or 0 (white), and a value of 1 may be assigned to essential genes and a value of 0 may be assigned to genes other than the essential genes.

FIG. 6C illustrates an example of a process of training a deep learning model. The training may be performed through a supervised learning method. In the supervised learning method, training data includes input data and label values. The input data may be N sets of gene expressions acquired through the same process as in FIG. 6A. The label value may utilize information already known experimentally as illustrated in FIG. 6B.

Essential gene information may be given as a label value (correct answer) that an output value of the deep learning model needs to have. The deep learning model may be a model that generates a value related to the probability of cell death when a specific set of gene expressions is input. The deep learning model may be trained so that the prediction result value (output value) outputs a value close to the actual value (correct answer value).

Hereinafter, the gene regulation network and deep learning model used by a researcher will be described.

**Example of Gene Regulation Network**

The above-described gene regulation network will be described.

A relationship of a target gene affecting expressions of other genes may be described by a network model. For example, a gene network model such as algorithm for the reconstruction of accurate cellular networks (ARACNe) describes a correlation between genes. Hereinafter, description will be made based on the ARACNe. A detailed description of the ARACNe construction process will be omitted. The gene network model may describe the relationship between genes a and b based on information on expressions of specific genes a and b. Assuming that P(a=on|b=on) represents the probability that the gene a is expressed when the gene b is expressed, when P(a=on|b=on)>P(b=on|a=on), then the gene b may be referred to as a regulatory gene of the gene a.

The expression relationship between genes may be identified in-silico using a network model representing the gene relationship. The network model representing the expression relationship of genes is referred to as a gene regulation network. The gene regulation network may identify genes affected by gene expression when the target gene to be evaluated is suppressed. Hereinafter, the gene regulation network will be described.

The gene regulation network simulates gene perturbation effects of CRISPR or RNAi in-silico. Therefore, the gene regulation network may be referred to as in-silico CRISPR or in-silico RNAi.

In the network model, the target gene has descendant genes that are affected by the target gene. The network model expresses, as an edge, the relationship between a node, which is a gene, and genes. Accordingly, the target gene may have not only a first sub-gene linked directly to the edge, but also a $j^{th}$ sub-gene linked through other nodes.

A relationship in which an expression of a certain gene affects expressions of other genes may be represented by Equation 1 below.

$$[\text{Equation 1}]$$

$$x_j^{'} = x_j - r_j \frac{y - y^{'}}{y} x_j$$

In Equation 1, Y denotes a target gene, and y denotes a default expression of a target gene of a cell. $X_j$ denotes the $j^{th}$ sub-gene of the target gene, and $x_j$ denotes the default expression of $X_j$. $r_j$ denotes a coefficient representing the correlation between the gene expressions of Y and $X_j$. y' denotes the perturbed gene expression of Y.

A researcher used the same transcriptome data as a reference sample for network construction. The CRISPR simulation was set to y'=0, and the RNAi simulation was set to y'=0.2y. Such a setting considers the results of previous studies.

The gene expression of the j$^{th}$ gene affected by a target gene i may be represented by a matrix P as in Equation 2 below.

[Equation 2]

$$P_{i,j} = -0.8(R \cdot B)_{i,j} + B_{j,j}$$

$$R = \begin{bmatrix} 1 & \cdots & r_n \\ \vdots & \ddots & \vdots \\ 0 & \cdots & 1 \end{bmatrix} \text{ and } B = \begin{bmatrix} x_1 & \cdots & 0 \\ \vdots & \ddots & \vdots \\ 0 & \cdots & x_n \end{bmatrix}$$

where

In Equation 2, R denotes a matrix representing an expression relationship. B denotes a default expression matrix filled with zeros except for diagonals.

To use the ARACNe, a researcher used a conditional probability instead of a correlation coefficient. The j$^{th}$ neighboring gene X$_j$ affected by the target gene Y may be expressed as a conditional probability as in Equation 3 below.

[Equation 3]

$$P(X_j = \text{activator}) = \frac{P(Y = \text{up} \cap X_j = \text{up}) + P(Y = \text{down} \cap X_j = \text{down})}{P(X_j = \text{up}) + P(X_j = \text{down})}$$

$$P(Y = \text{activator}) = \frac{P(X_j = \text{up} \cap Y = \text{up}) + P(X_j = \text{down} \cap Y = \text{down})}{P(Y = \text{up}) + P(Y = \text{down})}$$

$$P(X_j = \text{inhibitor}) = \frac{P(Y = \text{down} \cap X_j = \text{up}) + P(Y = \text{up} \cap X_j = \text{down})}{P(X_j = \text{up}) + P(X_j = \text{down})}$$

$$P(Y = \text{inhibitor}) = \frac{P(X_j = \text{down} \cap Y = \text{up}) + P(X_j = \text{up} \cap Y = \text{down})}{P(Y = \text{up}) + P(Y = \text{down})}$$

Up or down of the expression was determined based on a reference transcriptome sample used for the network construction. Each gene has an average expression $\mu$ and a standard deviation expression $\sigma$ determined from the reference sample.

When the expression of X$_j$ and Y in the reference sample is greater than $\mu+\sigma$, the researcher set X$_j$=up and Y=up. On the other hand, when the expressions of X$_j$ and Y in the reference sample were less than $\mu+\sigma$, the researcher set X$_j$=down and Y=down.

When the target gene Y and sub-gene X$_j$ have the relationship "P(X$_j$=activator)+P(Xj=inhibitor)<P(Y=activator)+P(Y=inhibitor),", X$_j$ may be the regulatory target of Y. The link relationship (up or down) between X$_j$ and Y may be determined by comparing P(Y=activator) and P(Y=inhibitor).

Expression X'$_j$ of X$_j$ that is affected by the perturbed expression of Y can be defined as in Equation 4 below.

[Equation 4]

$$x_j' = \begin{cases} x_j - P(Y = \text{activator})\dfrac{y - y'}{y}x_j, & \text{if } P(Y = \text{activator}) > P(Y = \text{inhibitor}) \\ x_j + P(Y = \text{inhibitor})\dfrac{y - y'}{y}x_j, & \text{if } P(Y = \text{activator}) < P(Y = \text{inhibitor}) \end{cases}$$

**Example of** Process **of Constructing** Deep Learning Model

The process of constructing the above-described deep learning model will be described. The deep learning model may be implemented in various structures. The researcher constructed models by adjusting (i) parameters for the model structure, such as the number of hidden layers and the number of hidden nodes, (ii) parameters for the model algorithm, such as training rate, momentum, batch size, activation function, and initial weight distribution, and (iii) regularization parameters L1 and L2, and parameters to solve overfitting problems such as dropout rate.

The researcher used a model of a stacked denoising autoencoder (SdA) structure. However, the output layer used the same number of nodes as the input layer.

The researcher generated a stochastically corrupted version of the input vector x, which includes the expressions of perturbed n genes by using a process known as denoising. $x \in [0,1]^n$. SdA maps the corrupted x to the hidden layer y using the activation function f. $y \in [0,1]^m$. Such an encoding process may be represented by Equation 5 below.

[Equation 5]

$$y = f(Wx + b)$$

W denotes a weight matrix, and b denotes bias.

A vector z reconstructed through a decoding process may be represented as in Equation 6 below. The decoding is performed in a way that minimizes the cost represented by the reconstruction error.

[Equation 6]

$$z = f\left(W^T y + b'\right)$$

The cost may be defined differently depending on the type of activation function. Equation 7 below is the cost for the ReLU function, and Equation 8 below is the cost for the sigmoid function.

[Equation 7]

$$\text{Cost} = \frac{1}{B} \sum_{k=1}^{B} \left(x_k - z_k\right)^2$$

[Equation 8]

$$\text{Cost} = -\frac{1}{B} \sum_{k=1}^{B} \left[x_k \, \log z_k + (1 - x_k) \, \log(1 - z_k)\right]$$

B denotes the batch size. Some values of the input vector x are masked according to the dropout rate. A parameter θ (weight and bias) is updated for each training course according to stochastic gradient descent. The updated parameter may be represented as in Equation 9 below.

[Equation 9]

$$\theta_{t+1} = \theta_t - \alpha \nabla_{\theta_t}$$

t denotes a training epoch.

After the initial training process, the researcher optimized a loss function represented by Equation 10 below.

[Equation 10]

$$\text{Loss} = \text{NLL} + \lambda_1 ||w||_1 + \lambda_2 ||w||_2$$

NLL is an average of negative log likelihood. $\lambda 1||w||_1 + \lambda 2||w||_2$ is a regularization term of an elastic net. $||\cdot||_p$ is the $L_p$ norm represented by Equation 11 below.
[Equation 11]

$$||w||_p = \left( \sum_{j=0}^{|w|} |w_j|^p \right)^{\frac{1}{p}}$$

$\lambda_p$ denotes a hyperparameter that controls the relative contribution of each regularization item. The elastic net was known to have better performance than the case of using $L_1$ or $L_2$ alone. The NLL($\theta$) of the loss function may be represented by Equation 12 below.

[Equation 12]

$$NLL(\theta) = -\frac{1}{B} \sum_{i=1}^{B} \left( Y^i \log f(\theta)^i + \left(1-Y^i\right) \log\left(1-f(\theta)^i\right) \right)$$

$f(\theta)^i$ is the gene expression of the target gene i in a mini batch size B. Each target Y may have a value of 0 or 1. 1 indicates that Y is an essential gene in the cell. The parameters of the loss function are updated through an inverse algorithm along with the momentum. The momentum for the loss function may be represented by Equation 13 below.
[Equation 13]

$$\theta_{t+1} = \theta_t + v_{t+1},$$

$$v_{t+1} = \mu v_t - \varepsilon \nabla \left( \text{Loss}\left(\theta^t\right) \right)$$

$\varepsilon$ denotes the training rate, $\mu$ denotes the momentum coefficient, and $\nabla(\text{Loss}(\theta^t))$d denotes a slope at $\theta^t$. $v_0$ is set to 0.

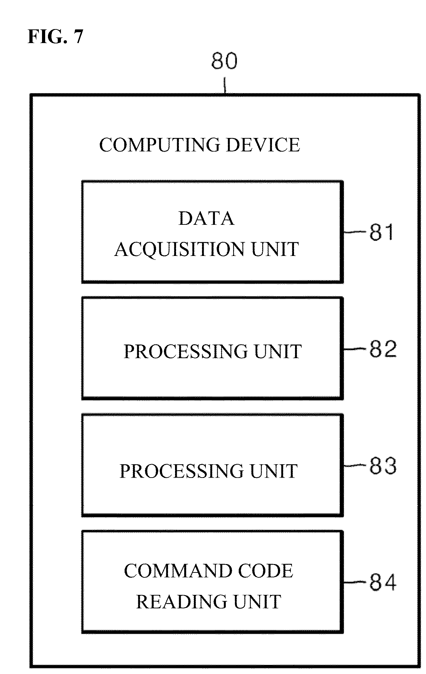

FIG. 7 illustrates an example of a computing device 80 for predicting essential genes of a cell using a deep learning model.
The computing device 80 is configured to determine essential genes of tumor cells using a deep learning model that receives expressions of cellular genes and outputs a probability of cell death. The cell may be a tumor cell or a normal cell.
The computing device 80 may include a data acquisition unit 81 configured to acquire information on the deep learning model and information on one or more gene regulation networks.
The computing device 80 may include a processing unit 82.
The computing device 80 may include a command code reading unit 84 that reads command codes executed by the processing unit 82 from a storage unit 83 which is accessible by the computing device.
The storage unit 83 may be provided inside or outside the computing device 80 and may be accessible by the computing device 80 through a network.
The processing unit 82 may execute the command codes to output a result value for an input value of the received sample.
Furthermore, a computer-readable non-transitory recording medium may be provided in which command codes for determining essential genes of a cell using a deep learning model that receives expressions of cellular genes and outputs a probability of cell death are recorded. Each command code performs the process of pre-processing (gene expression perturbation) the above-described input data and outputting essential genetic information predicted by inputting the input value to the deep learning model, in the computer device in which the corresponding code operates.

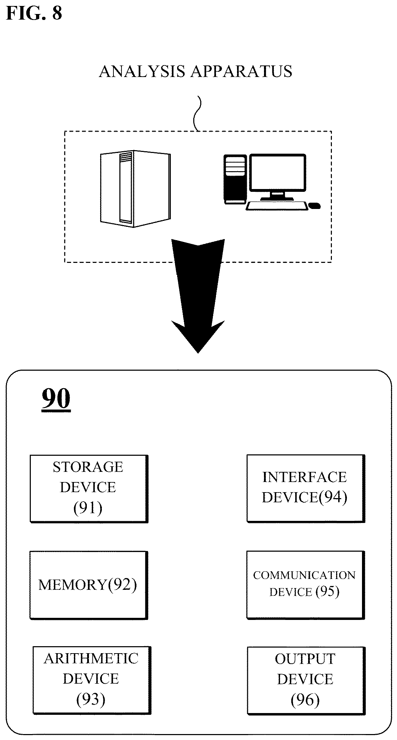

FIG. 8 illustrates an example of an analysis apparatus for identifying an essential gene. An analysis apparatus 90 is an apparatus corresponding to the analysis apparatus 12 or 13 of FIG. 1.

The analysis apparatus 90 may be physically implemented in various forms. For example, the analysis apparatus 90 may have the form of a computer device such as a PC, a server of a network, an image processing-only chipset, or the like. The computer device may include a mobile device such as a smart device.

The analysis apparatus 90 may include a storage device 91, a memory 92, an arithmetic device 93, an interface device 94, a communication device 95, and an output device 96.

The storage device 91 stores a deep learning model for predicting essential genes of a cell. The deep learning model needs to be trained in advance. The storage device 91 may store a gene expression perturbation program (gene regulation network) for perturbing a specific gene expression. Furthermore, the storage device 91 may store a program, a source code, or the like required for data processing. The storage device 91 may store input genome expression and predicted essential gene information.

The memory 92 may store data, information, and the like generated while the analysis apparatus 90 analyzes data.

The interface device 94 is a device that receives predetermined commands and data from an external device. The interface device 94 may receive genome expression data of a cell from a physically connected input device or external storage device. The interface device 94 may receive a learning model for data analysis. The interface device 94 may receive training data, information, and parameter values for training a learning model.

The interface device 94 may receive a selection command for a target gene to be analyzed from a user.

The communication device 95 means a configuration for receiving and transmitting predetermined information through a wired or wireless network. The communication device 95 may receive genome expression data of a cell from an external object. The communication device 95 may also receive data for training a model. The communication device 95 may transmit essential genetic information determined for the input cell to an external object.

The communication device 95 or the interface device 94 is a device that receives predetermined data or commands from an external device. The communication device 95 or the interface device 94 may be referred to as an input device.

The output device 96 is a device that outputs predetermined information. The output device 96 may output an interface necessary for a data processing process, an analysis result, and the like.

The arithmetic device 93 may regulate the expression of the target gene by using the program stored in the storage device 91.

The arithmetic device 93 may convert expression data of genes into the vector sequence described above. In this case, the vector sequence includes information on a gene sequence and information on expressions of each gene.

The arithmetic device 93 may input the cellular gene expression pattern regulated to the deep learning model and output whether a cell dies. The arithmetic device 93 inputs a vector of a gene expression pattern to the deep learning model to obtain a constant output value.

The arithmetic device 93 may predict whether the target gene is an essential gene of a cell based on the output information.

The arithmetic device 93 may generate expression pattern information in which an expression of a target gene is regulated for each of normal cells and tumor cells of the same sample. The arithmetic device 93 may calculate a first value by inputting expression pattern information on normal cells to the deep learning model. In addition, the arithmetic device 93 may calculate a second value by inputting expression pattern information on tumor cells to the deep learning model. When the first value indicates cell survival and the second value indicates cell death, the arithmetic device 93 may determine that the target gene is a specific essential gene of the tumor cells of the sample.

Meanwhile, the arithmetic device 93 may train a learning model used for essential gene prediction by using the given training data.

The arithmetic device 93 may be a device such as a processor, an AP, or a chip embedded with a program that processes data and processes a predetermined operation.

### Effect Verification Experiment

The results of verifying the effects of the above-described deep learning model will be described. The researcher used, as a reference, the result of calculating a dependency score for breast cancer patients among the results of the previous study. The dependence score refers to a quantitative value for a gene essential for breast cancer.

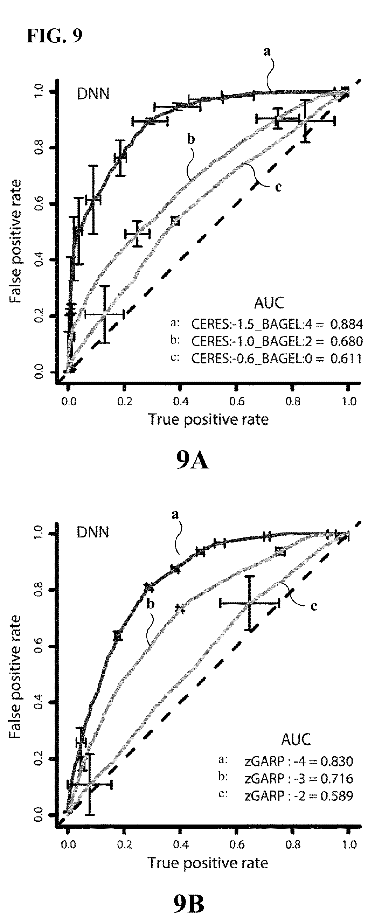

FIG. 9 illustrates an experimental result verifying an effect of a deep learning model.

The researcher merged and referenced the results of a CRISPR associated protein 9 (CRISPR-Cas9) screen of 28 breast cancer cell lines, which yield a dependency score, referred to as CERES, and 25 breast cancer cell lines, which yield a dependency score, referred to as BAGEL. The researcher divided references based on cutoff values of the CERES and BAGEL to show similar dependence for each cell line. A first reference a is CERES=-1.5+BAGEL=4. A second reference b is CERES=-1.0+BAGEL=2. A third reference (c) is CERES=-0.6+BAGEL=0. FIG. 9A illustrates a receiver operating characteristic (ROC) curve by comparing the results predicted by the above-described deep learning model with the reference. FIG. 9A is an example of generating a gene expression pattern by a gene perturbation method based on in-silico CRISPR and inputting the generated gene expression pattern to the deep learning model. An area under curve (AUC) for the first reference was 0.884, an AUC for the second reference was 0.680, and an AUC for the third reference was 0.611.

In addition, the researcher used, as a reference, short hairpin (shRNA) dropout screen results for 77 breast cancer cell lines in the previous study. As a result of this experiment, a regularized gene activity ranking profile (GARP) score was derived for each gene. This score is also referred to as zGARP The researcher used three cutoff values (zGARP=-2, -3, or -4). FIG. 9B illustrates an ROC curve by comparing the results predicted by the above-described deep learning model with the reference. FIG. 9A is an example of generating a gene expression pattern by a gene perturbation method based on in-silico RNAi and inputting the generated gene expression pattern to the deep learning model. The AUC for the reference a set to zGARP as -4 was 0.830, the AUC for the reference b set to zGARP as -3 was 0.716, and the AUC for the reference c set to zGARP as -2 was 0.589.

In addition, the cell-specific essential gene identification method or tumor-specific essential gene identification method as described above may be implemented as a program (or application) including an executable algorithm that may be executed in a computer. The program may be stored and provided in a non-transitory computer-readable medium.

The non-transitory computer-readable medium is not a medium that stores data therein for a while, such as a register, a cache, a memory, or the like, but means a medium that semi-permanently stores data therein and is readable by an apparatus. Specifically, various applications or programs described above may be provided by being stored in non-transitory readable media such as a compact disk (CD), a digital video disk (DVD), a hard disk, a Blu-ray disk, a universal serial bus (USB), a memory card, a read-only memory (ROM), a programmable read only memory (PROM), an erasable PROM (EPROM), an electrically EPROM (EEPROM), or a flash memory.

The transitory readable media refer to various RAMs such as a static RAM (SRAM), a dynamic RAM (DRAM), a synchronous DRAM (SDRAM), a double data rate SDRAM (DDR SDRAM), an enhanced SDRAM (ESDRAM), a synclink DRAM (SLDRAM), and a direct rambus RAM (DRRAM).

The present embodiment and the drawings attached to the present specification only clearly show some of the technical ideas included in the above-described technology, and therefore, it will be apparent that all modifications and specific embodiments that can be easily inferred by those skilled in the art within the scope of the technical spirit included in the specification and drawings of the above-described technology are included in the scope of the above-described technology.

**Claims**

1. A machine learning model-based essential gene identification method comprising:

    receiving, by an analysis apparatus, expression pattern information on genes of a specific cell;
    inputting, by the analysis apparatus, the expression pattern information to a machine learning model; and
    determining, by the analysis apparatus, whether a target gene among the genes is essential in survival of the cell on the basis of information output by the machine learning model,
    wherein the machine learning model includes a parameter trained based on a training data set, and the training data set includes data for a gene expression of the specific call and a label value for whether the specific cell dies.

2. The machine learning model-based essential gene identification method of claim 1, wherein the expression pattern information is information in which an expression of the target gene is changed, and
    the machine learning model-based essential gene identification method further includes generating, by the analysis apparatus, the expression pattern information by changing the expression of the target gene from information on an initial expression on the genes of the specific cell.

3. The machine learning model-based essential gene identification method of claim 2, wherein the analysis apparatus generates the expression pattern information by determining expressions of the genes of the specific cell predicted when the expression of the target gene is constantly knocked-down using a gene regulation network.

4. The machine learning model-based essential gene identification method of claim 1, wherein data for a gene expression of the training data set is the gene expression of the specific cell measured experimentally, and the label value is a value for whether the specific cell having the gene expression dies.

5. The machine learning model-based essential gene identification method of claim 1, wherein the data for the gene expression of the training data set is expression data of the genes of the specific cell predicted when an expression of a specific gene is knocked-down using a gene regulation network, and the label value is a value for whether a cell observed experimentally dies when the expression of the specific gene is knocked-down or inhibited.

6. A machine learning model-based tumor cell-specific essential gene identification method comprising:

receiving, by the analysis apparatus, data for a gene expression of each of a normal cell and a tumor cell of the same target;
inputting, by the analysis apparatus, first gene expression pattern information, in which an expression of a target gene to be analyzed is regulated for the tumor cell, to a machine learning model to generate a first value;
inputting, by the analysis apparatus, second gene expression pattern information, in which an expression of the same gene as the target gene is regulated for the normal cell, to the machine learning model to generate a second value; and
comparing, by the analysis apparatus, the first value with the second value to determine whether the target gene is an essential gene specific to the tumor cell,
wherein the machine learning model includes a parameter trained based on a training data set, and the training data set includes data for gene expression of the specific call and a label value for whether a specific cell dies.

7. The machine learning model-based tumor cell-specific essential gene identification method of claim 6, further comprising performing, by the analysis apparatus, pre-processing for regulating the expression of the target gene to be analyzed among the data for the gene expression of each of the normal cell and the tumor cell.

8. The machine learning model-based tumor cell-specific essential gene identification method of claim 6, further comprising generating, by the analysis apparatus, the first gene expression pattern information and the second gene expression pattern information including expressions of genes predicted when the expression of the target gene is constantly knocked-down using a gene regulation network for each of the normal cell and the tumor cell.

9. The machine learning model-based tumor cell-specific essential gene identification method of claim 6, wherein the data for the gene expression of the training data set is a gene expression of a specific cell measured experimentally, and the label value is a value for whether the specific cell having the gene expression dies.

10. The machine learning model-based tumor cell-specific essential gene identification method of claim 6, wherein the data for the gene expression of the training data set is expression data of the genes of the specific cell predicted when an expression of a specific gene is knocked-down using a gene regulation network, and the label value is a value for whether a cell observed experimentally dies when the expression of the specific gene is knocked-down or inhibited.

11. The machine learning model-based tumor cell-specific essential gene identification method of claim 6, wherein the analysis apparatus determines that the target gene is an essential gene specific to the tumor cell when the first value indicates death of the tumor cell and the second value indicates survival of the normal cell.

12. An analysis apparatus for selecting a machine learning model-based essential gene, comprising:

an input device configured to receive expression data for cellular genes;
a storage device configured to store a machine learning model that receives a gene expression pattern in which an expression of a specific gene is regulated and outputs essentiality information on the specific gene; and
a processor configured to input a gene expression pattern for the cell, in which an expression of a target gene is regulated in the expression data input from the input device, to the machine learning model, and determine essentiality of the target gene based on a value output by the machine learning model,
wherein the machine learning model includes a parameter determined based on a training data set, and the training data set includes data for a gene expression of the specific call and a label value for whether the specific cell dies.

13. The analysis apparatus of claim 12, wherein the storage device further includes a gene regulation network, and the processor generates the gene expression pattern of the cell predicted when the expression of the target gene is constantly knocked-down by using the gene regulation network.

14. The analysis apparatus of claim 12, wherein the input device receives expression data of genes for the tumor cell, and the processor inputs the gene expression pattern for the tumor cell to the machine learning model to calculate a first value and to determine whether the target gene of the tumor cell is essential.

15. The analysis apparatus of claim 14, wherein the input device receives the expression data of the genes for the normal cell, and

   the processor inputs the gene expression pattern for the normal cell to the machine learning model to calculate a second value, and
   determines that the target gene is an essential gene specific to the tumor cell when the first value indicates death of the tumor cell and the second value indicates survival of the normal cell.

16. The analysis apparatus of claim 12, wherein an arithmetic device converts the gene expression pattern into a vector and inputs the vector to the machine learning model, and
   the vector includes an order of a gene sequence and information on an expression of each gene.

FIG. 1

**FIG. 2**

**20**

| INPUT SAMPLE GENOME EXPRESSION PATTERN (21) | → | REGULATE SPECIFIC GENE EXPRESSION TO BE EVALUATED (22) | GENE EXPRESSION PATTERN WITH REGULATED EXPRESSION → | ANALYZE BASED ON DEEP LEARNING MODEL (23) | ANALYSIS RESULT → |

SELECT GENE TO BE EVALUATED

## FIG. 3

**3A**

**3B**

**3C**

## FIG. 4

4A

4B

4C

FIG. 5

5A

5B

5C

# FIG. 6

GENE 1
GENE K
GENE EXPRESSION
SUPRESSION
EXPERIMENT
GENE 2
GENE N
GENE 3

GENE 1
GENE K
GENE 2
GENE N
GENE 3

GENE EXPRESSION
KNOCKDOWN
PERTURBATION
SIMULATION

GENE1 100
GENE2 50
GENE3 10
GENE K 3
GENE N 100

TRANSCRIPTOME
OF CELL LINE 810

0
50
5
3
120

812

813
100
50
10
0
100

**6A**

GENE1 1 (ESSENTIALGENE)
GENE2 0
GENE3 1 (ESSENTIALGENE)
GENE K 0
GENE N 0

ACTUAL VALUE

**6B**

SIMULATION
TRANSCRIPTOME
VALUE

0
50
5
3
120

INPUT LAYER/ HIDDEN LAYER/ HIDDEN LAYER/ OUTPUT LAYER

CELL DEATH
CELL SURVIVAL

TRAIN ESSENTIAL GENE
PREDICTION MODEL

**6C**

RESULT VALUE

ACTUAL VALUE

CORRECT ERROR

ADJUST BACKPROPAGA-TION WEIGHT

**FIG. 7**

80

COMPUTING DEVICE

DATA
ACQUISITION UNIT 81

PROCESSING UNIT 82

PROCESSING UNIT 83

COMMAND CODE
READING UNIT 84

**FIG. 8**

ANALYSIS APPARATUS

**90**

| STORAGE DEVICE (91) | INTERFACE DEVICE(94) |
|---|---|
| MEMORY(92) | COMMUNICATION DEVICE (95) |
| ARITHMETIC DEVICE (93) | OUTPUT DEVICE (96) |

FIG. 9

**9A**

**9B**

## INTERNATIONAL SEARCH REPORT

| International application No. |
| --- |
| **PCT/KR2020/008843** |

**A. CLASSIFICATION OF SUBJECT MATTER**

**G16B 40/00**(2019.01)i; **G16B 25/10**(2019.01)i; **G16B 50/00**(2019.01)i; **G16H 20/00**(2018.01)i

According to International Patent Classification (IPC) or to both national classification and IPC

**B. FIELDS SEARCHED**

Minimum documentation searched (classification system followed by classification symbols)

G16B 40/00; G16B 25/10; G16B 50/00; G16H 20/00

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 발현 패턴(experssion pattern), 기계학습(mechine learning), 딥러닝(deep learning), 사멸 세포(apoptosis cell), 필수 유전자(essential gene)

**C. DOCUMENTS CONSIDERED TO BE RELEVANT**

| Category* | Citation of document, with indication, where appropriate, of the relevant passages | Relevant to claim No. |
| --- | --- | --- |
| A | CAMPOS, Tílio L. et al. An Evaluation of Machine Learning Approaches for the Prediction of Essential Genes in Eukaryotes Using Protein Sequence-Derived Features. Computational and Structural Biotechnology Journal. 2019, vol. 17, pp. 785-796 (published online on 08 June 2019). See abstract and pages 786-794. | 1-16 |
| A | TIAN, David et al. Identifying mouse developmental essential genes using machine learning. Disease Models & Mechanisms. 2018, vol. 11, dmm064546. See abstract and pages 10-11. | 1-16 |
| A | ZHANG, Xue et al. Predicting Essential Genesand Proteins Based on Machine Learning and Network Topological Features: A Comprehensive Review. Frontiers in Physiology. 2016, vol. 7, Article 75. See abstract and pages 2-9. | 1-16 |
| A | CHEN, Lei et al. Prediction and analysis of essential genes using the enrichments of gene ontology and KEGG pathways. PLOS One. 2017, vol. 12, no. 9, e0184129. See entire document. | 1-16 |
| A | IJZENDOORN, David G. P. et al. Machine learning analysis of gene expression data reveals novel diagnostic and prognostic biomarkers and identifies therapeutic targets for soft tissue sarcomas. PLOS Computational Biology. 2019, vol. 15, no. 2, e1006826 (published on 20 February 2019). See entire document. | 1-16 |

☐ Further documents are listed in the continuation of Box C.  ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **21 October 2020** | **21 October 2020** |

| Name and mailing address of the ISA/KR | Authorized officer |
| --- | --- |
| **Korean Intellectual Property Office**<br>**Government Complex Daejeon Building 4, 189, Cheongsa-ro, Seo-gu, Daejeon, Republic of Korea**<br>**35208** | |
| Facsimile No. **+82-42-481-8578** | Telephone No. |

Form PCT/ISA/210 (second sheet) (July 2019)